# FASCICULE DE BREVET EUROPEEN

(11) **EP 2 986 613 B1**
(45) Date de publication et mention de la délivrance du brevet: **30.05.2018**
(21) Numéro de dépôt: 14725221.7
(22) Date de dépôt: 15.04.2014
(51) Int. Cl.: C07D 491/22, G01N 24/08

(54) **COMPOSES BIRADICALAIRES DU TYPE DINITROXYDE RIGIDES UTILISES COMME AGENTS DE POLARISATION PERFECTIONNES POUR LES TECHNIQUES DE POLARISATION DYNAMIQUE NUCLEAIRE**
STARRE BIRADIKALE DINITROXIDVERBINDUNGEN ALS VERBESSERTE POLARISIERENDE MITTEL FÜR DYNAMISCHE NUKLEARE POLARISATIONSTECHNIKEN
RIGID DINITROXIDE BIRADICAL COMPOUNDS USED AS IMPROVED POLARISING AGENTS FOR DYNAMIC NUCLEAR POLARISATION TECHNIQUES

(30) Priorité: 15.04.2013 FR 1353399
(43) Date de publication de la demande: 24.02.2016
(73) Titulaire: Université d'Aix-Marseille, 13007 Marseille (FR); Centre National de la Recherche Scientifique (C.N.R.S.), 75016 Paris (FR); Bruker Biospin, 67160 Wissembourg (FR); Ecole Normale Supérieure de Lyon, 69007 Lyon (FR); ETH Zürich, 8092 Zürich (CH); Université Claude Bernard Lyon 1, 69100 Villeurbanne (FR)
(72) Inventeur: OUARI, Olivier, F-13008 Marseille (FR); TORDO, Paul, F-13010 Marseille (FR); CASANO, Gilles, F-13110 Port De Bouc (FR); ROSAY, Mélanie, Bedford, Massachusetts 01730 (US); AUSSENAC, Fabien, F-67500 Haguenau (FR); COPERET, Christophe, CH-8037 Zurich (CH); LESAGE, Anne, F-69140 Rillieux La Pape (FR); ROSSINI, Aaron, F-69100 Villeurbanne (FR); EMSLEY, Lyndon, F-38950 Saint Martin Le Vinoux (FR); ZAGDOUN, Alexandre, F-69007 Lyon (FR)
(74) Mandataire: Cabinet Becker et Associés
(86) Numéro de dépôt international: PCT/FR2014/050920
(87) Numéro de publication internationale: WO 2014/170601

(56) Documents cités:
- WO-A1-02/48205
- WO-A1-2010/108995
- MATTHEW K. KIESEWETTER ET AL: "Dynamic Nuclear Polarization with a Water-Soluble Rigid Biradical", JOURNAL OF THE AMERICAN CHEMICAL SOCIETY, vol. 134, no. 10, 14 mars 2012 (2012-03-14), pages 4537-4540, XP055077533, ISSN: 0002-7863, DOI: 10.1021/ja212054e
- ERIC L. DANE ET AL: "Rigid Orthogonal Bis-TEMPO Biradicals with Improved Solubility for Dynamic Nuclear Polarization", THE JOURNAL OF ORGANIC CHEMISTRY, vol. 77, no. 4, 17 février 2012 (2012-02-17), pages 1789-1797, XP055077525, ISSN: 0022-3263, DOI: 10.1021/jo202349j
- ALEXANDRE ZAGDOUN ET AL: "A Slowly Relaxing Rigid Biradical for Efficient Dynamic Nuclear Polarization Surface-Enhanced NMR Spectroscopy: Expeditious Characterization of Functional Group Manipulation in Hybrid Materials", JOURNAL OF THE AMERICAN CHEMICAL SOCIETY, vol. 134, no. 4, 1 février 2012 (2012-02-01), pages 2284-2291, XP055077530, ISSN: 0002-7863, DOI: 10.1021/ja210177v
- YSACCO ET AL: "Properties of dinitroxides for use in dynamic nuclear polarization (DNP)", PHYSICAL CHEMISTRY CHEMICAL PHYSICS, ROYAL SOCIETY OF CHEMISTRY, CAMBRIDGE, GB, vol. 12, no. 22, 10 mai 2010 (2010-05-10), pages 5841-5845, XP002711482, ISSN: 1463-9076, DOI: 10.1039/C002591G

## Description

### DOMAINE TECHNIQUE DE L'INVENTION

La Résonance Magnétique Nucléaire (RMN) découverte il y a plus de soixante ans, est aujourd'hui la technique d'analyse de la matière la plus polyvalente et la plus performante dans de très nombreux domaines, notamment en chimie moléculaire et macromoléculaire, biochimie, science des matériaux, IRM tissulaire ou fonctionnelle.

Toutefois, la RMN, en tant qu'outil de détection ou d'imagerie, est limitée par sa faible sensibilité intrinsèque. Cette faible sensibilité est due à la très faible différence d'énergie, et donc la très faible différence de population (désignée par polarisation de spin P_{I}) entre les états de spin nucléaire entre lesquels on observe le phénomène de résonance.

A l'équilibre thermique la polarisation de spin de l'électron (Ps) est beaucoup plus grande que celle des noyaux actifs en RMN. Dans les méthodes de la Polarisation Dynamique Nucléaire (PDN) l'irradiation par micro-ondes (MW) permet de transférer la polarisation de spin électronique (Ps) vers des noyaux dont on étudie la résonance magnétique. En pratique on incorpore dans l'échantillon à étudier par RMN, une substance paramagnétique (agent de polarisation) renfermant un (monoradical) ou plusieurs (polyradical) électrons célibataires. Dans l'échantillon, les polarisations de spin électronique et nucléaire étant corrélées *via* diverses interactions électron-noyau, l'irradiation (MW) du spectre de Résonance Paramagnétique Electronique (RPE) de l'agent de polarisation à une fréquence appropriée, entraîne un transfert de polarisation des spins électroniques vers les spins des noyaux étudiés et il en résulte une amplification des signaux RMN de ces derniers.

L'invention porte sur des biradicaux organiques utilisés comme agents de polarisation dans les techniques de Polarisation Dynamique Nucléaire (PDN).

Au cours des dernières années, l'introduction d'agents polarisants de plus en plus efficaces en PDN a conduit à une augmentation continue des facteurs d'amplification, ε (ε = I_{signal RMN avec MW}/I_{signal RMN sans MW}), des signaux RMN des noyaux polarisés. Grâce au gain de sensibilité spectaculaire fourni par la PDN, des caractéristiques structurales jusqu'alors inaccessibles ont été obtenues, *via* le couplage de la PDN avec la RMN à l'angle magique (PDN/MAS RMN), sur des fibrilles, des protéines membranaires, des capsides virales, des assemblages de cellules entières. En 2010, l'équipe de L. Emsley a introduit la Spectroscopie RMN de surface exaltée par Polarisation Dynamique Nucléaire à haut champ magnétique (DNP SENS). Par exemple, sur des silices mésoporeuses fonctionnalisées par des unités phénols (0.5 µmol d'unités phénols/mg de matériau) la DNP SENS a permis l'acquisition du spectre RMN CPMAS du ¹³C en abondance naturelle en seulement 0.5 h. Tous les carbones de l'unité phénol ont pu être caractérisés ainsi que des sous-produits présents dans la matrice. Le gain de sensibilité a permis l'acquisition en quelques minutes de spectres HETCOR 1H-13C.

### ETAT DE LA TECHNIQUE ANTERIEURE

Dans ce domaine, on connaît du document US 2005/107696 des agents de polarisation utilisés pour amplifier les signaux en RMN et en IRM via une polarisation dynamique nucléaire (DNP). Les agents de polarisation comprennent deux ou plusieurs centres paramagnétiques, de préférence deux centres paramagnétiques. Dans un mode de réalisation préféré, l'agent de polarisation est un biradical (dinitroxyde) qui comprend deux unités nitroxydes attachées par une chaîne polyéthylène glycol de longueur variable.

Les dinitroxydes décrits dans cette demande sont reliés par un lien de jonction non rigide. De ce fait, la polarisation n'est pas suffisamment optimisée. Il en est de même pour le dinitroxyde TOTAPOL, qui est également divulgué dans l'art antérieur.

Pour pallier ces inconvénients, la demande WO 2010/108995 propose des composés biradicalaires du type dinitroxyde, qui présentent entre les deux unités nitroxyde une jonction rigide pouvant être d'un nombre impair de liaisons spiraniques, maintenant une orientation et une distance particulières entre les deux unités nitroxydes. Cette demande divulgue plusieurs composés dont le bTbK (bis-TEMPO-bis-Ketal), qui a montré une nette amélioration de la polarisation.

### EXPOSE DE L'INVENTION

L'invention vise notamment à proposer de nouveaux composés biradicalaires du type dinitroxyde, où les deux unités nitroxydes sont maintenues par une jonction rigide, et qui montrent une plus grande efficacité par rapport à l'art antérieur.

Ainsi, l'invention porte sur un composé biradicalaire du type dinitroxyde, où les deux unités nitroxydes sont maintenues par une jonction rigide, de formule générale (I): 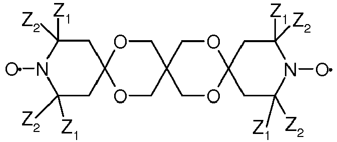 dans laquelle
- Z1 et Z2, combinés ensemble avec le même atome de carbone du cycle nitroxyde auquel ils sont liés, forment un spirocyclohexyle substitué par un ou deux groupements R3, R3 étant un groupe phényle éventuellement substitué par un ou plusieurs groupements R4, R4 étant un phényle.

Avantageusement, le composé biradicalaire est marqué isotopiquement, de préférence par l'un parmi le Deutérium (²H), le Carbone 13 (¹³C) et l'Azote 15 (¹⁵N).

Avantageusement, le composé biradicalaire est choisi parmi le bPhCTbK, le TEKPol 2 et le TEKPol 3, ou un dérivé de ceux-ci substitué sur un ou plusieurs des groupements phényles par un groupement de type R4 tel que défini ci-avant.

Le bPhCTbK, ou encore appelé TEKPol, est le bis-PhénylCyclohexylTEMPO-bis-Ketal, c'est-à-dire le 22,41-dinitroxyl-3,19,26,38-tetraphenyl-22,41 diazaheptaspiro [5.1.2.2.1.5.1.5.1.2.2.1.5.1] hentetracontane.

Le TEKPol 2 est le 22,41-dinitroxyl-2,4,18,20,25,27,37,39-octaphenyl-22,41 diazaheptaspiro [5.1.2.2.1.5.1.5.1.2.2.1.5.1] hentetracontane.

Le TEKPol 3 est le 22,41-dinitroxyl-3,19,26,38-tetra-p-biphenyl-22,41 diazaheptaspiro [5.1.2.2.1.5.1.5.1.2.2.1.5.1] hentetracontane.

L'invention porte également sur l'utilisation du composé biradicalaire comme agent de polarisation pour la mise en oeuvre de techniques de Résonance Magnétique Nucléaire, dans les techniques de physique des particules ainsi que dans les techniques d'imagerie médicale.

Il est également divulgué un composé paramagnétique comprenant au moins une unité biradicalaire de type dinitroxyde tel que décrite précédemment.

L'invention porte également sur une composition comprenant au moins un composé biradicalaire de type dinitroxyde tel que décrit précédemment.

Un autre objet de l'invention consiste en un procédé de polarisation d'un échantillon comprenant une étape de mise en contact de l'échantillon avec un agent de polarisation, dans lequel l'agent de polarisation est un composé biradicalaire de type dinitroxyde tel que décrit précédemment.

L'invention porte également sur un procédé d'analyse dans des techniques de biologie structurale, de Résonance Magnétique Nucléaire du solide ou appliquées à des échantillons liquides, de physique des particules ou d'imagerie médicale, comprenant une polarisation d'un échantillon dans laquelle l'échantillon est mis en contact avec un agent de polarisation, dans lequel l'agent de polarisation est un composé biradicalaire de type dinitroxyde tel que décrit précédemment.

### BREVE DESCRIPTION DES FIGURES

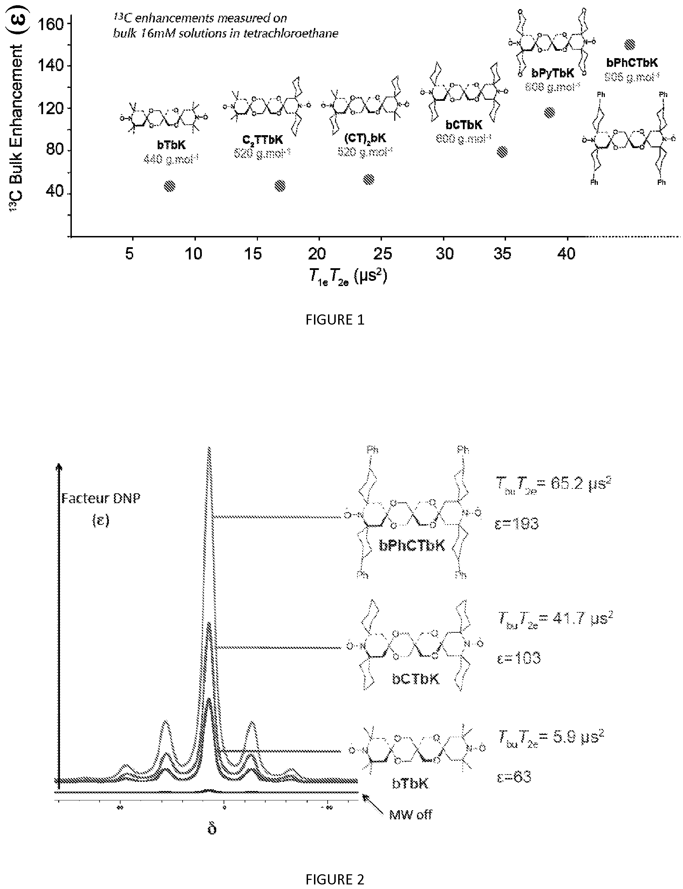

D'autres caractéristiques, détails et avantages de l'invention ressortiront à la lecture de la description qui suit, en référence aux figures 1 et 2 annexées, qui illustrent l'augmentation par la PDN du signal RMN du ¹³C du solvant, pour différents biradicaux dissous dans les solutions de tétrachloroéthane.

Les figures 1 et 2 montrent l'évolution du facteur d'amplification par la PDN du signal RMN du ¹³C du tétrachloroéthane utilisé comme solvant en présence de divers dinitroxydes rigides dont le bTbK, le bCTbK et le bPhCTbK.

### DESCRIPTION DETAILLEE D'UN MODE DE REALISATION

Des essais ont montré que, dans du 1,1,2,2 tétrachloroéthane à environ 100 K, le bCTbK (bis-CyclohexyITEMPO-bis-Ketal), présente une valeur T₁ₑ qui est 1,7 fois plus longue que celle du bTbK. Sur un modèle hybride de matériau à base de silice mésoporeuse, l'amplification des signaux RMN observée avec le bCTbK est 2,5 fois supérieure à celle obtenue avec le bTbK. (J. Am. Chem. Soc., 2012, 134, 2284)

Selon le même principe, la demanderesse a développé des dérivés de plus en plus volumineux du bTbK qui sont solubles dans les solvants organiques.

Selon un mode de réalisation de l'invention, la figure 1 représente l'évolution à 100 K du facteur d'amplification (ε) par la PDN, du signal RMN du ¹³C en abondance naturelle du tétrachloroéthane, en présence de divers dinitroxydes comme agent polarisant. De même la Figure 2 représente le facteur d'amplification (ε) par la PDN, du signal RMN du 13C en abondance naturelle du tétrachloroéthane, en présence de divers dinitroxydes possédant des T1e et T2e différents comme agent polarisant. Les expériences ont été effectuées sur un spectromètre Bruker Avance III 400 MHz DNP MAS RMN équipé d'un gyrotron émettant des micro-ondes de 263 GHz de fréquence.

La concentration en dinitroxyde est de 16 mM et la fréquence de rotation du rotor est de 8 kHz.

Comme l'illustrent les figures 1 et 2 annexées et le tableau 1 ci-après, des relations fortes sont observées entre le poids moléculaire de ces agents polarisants, leurs temps de relaxation transversal et longitudinal T1e et T2e qui en dépendent, et surtout leur efficacité (ε) en PDN.

| **Composé** | **Structure** | **Poids Moléculaire** | **ε_{C CP}^{a}** | ***T*₁ₑ^{b}** | ***T₂ₑ*^{c}** |
|---|---|---|---|---|---|
| | | **(g.mol⁻¹)** | | **(µs)** | **(ns)** |
| **bTbK** | 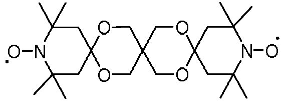 | 440 | 47 | 10 | 588 |
| **bCTbK** | 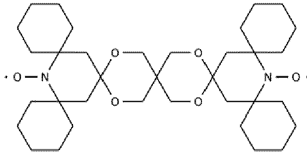 | 600 | 80 | 37 | 1128 |
| **bPyTbK** | 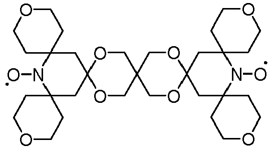 | 608 | 115 | 32 | 1160 |
| **bPhCTbK** | 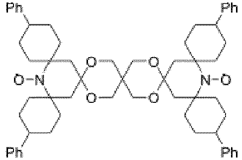 | 904.5 | 150 | 47 | 1416 |

Tableau 1. a) Facteur d'amplification (ε) du signal de RMN du 13C en abondance naturelle du tétrachloroéthane. Les conditions expérimentales sont identiques à celles mentionnées dans les Figures 1 et 2. b) Temps de relaxation électronique longitudinal (à 100 K, 95 GHmz). c) Temps de relaxation électronique transversal (à 100 K, 95 GHz)

En particulier, en utilisant le bPhCTbK (bis-PhénylCyclohexylTEMPO-bis-Ketal) comme agent de polarisation, les facteurs d'amplification des signaux RMN obtenus sont au moins trois fois plus grands que ceux évoqués précédemment, avec le bTbK.

Ainsi un composé préféré est le bPhCTbK, de formule générale : 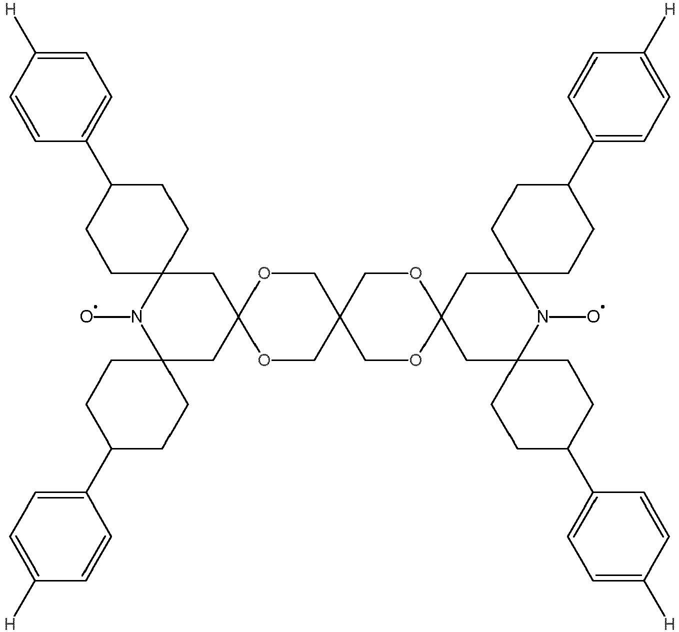 (22,41-dinitroxyl-3,19,26,38-tetraphenyl-22,41 diazaheptaspiro [5.1.2.2.1.5.1.5.1. 2.2.1. 5.1] hentetracontane).

Comme on peut le voir sur les Figures 1 et 2, les agents de polarisation actuellement les plus efficaces pour les applications DNP RMN MAS (rotation à l'angle magique - magic angle spinning) dans des solvants organiques sont, dans un ordre croissant d'efficacité, le bTbK, le bCTbK, et le bPyTbK.

Le bPhCTbK proposé par l'invention est encore plus efficace que l'agent le plus performant bPyTbK.

Cette efficacité peut être corrélée par la conception de dinitroxydes non seulement possédant les unités nitroxydes maintenues par une jonction rigide, mais aussi avec des temps de relaxation électronique longitudinal et /ou transversal (T1e, T2e) plus longs, par exemple avec la présence de quatre groupements aromatiques comme on peut le voir dans les formules générales.

Ainsi, cette efficacité du bPhCTbK peut se retrouver dans tous les composés revendiqués. En tout état de cause, un composé donné parmi ceux revendiqués serait, de la même manière, plus efficace qu'un composé analogue où les unités nitroxydes composés ne sont pas maintenues par une jonction rigide et/ou qui ne présente pas des groupements aromatiques.

En outre, l'utilisation d'un ou plusieurs substituants R4 permettrait d'accroître encore plus l'efficacité du processus de DNP et/ou ouvrir la possibilité d'utiliser la technique dite de RMN MAS DNP à plus haute température (150 - 200 K). En outre, ces substituants R4 peuvent être choisis pour améliorer la solubilisation du composé dans une solution donnée ou faciliter l'adjonction d'autres molécules.

De nombreuses combinaisons peuvent être envisagées sans sortir du cadre de l'invention ; l'homme du métier choisira l'une ou l'autre en fonction des contraintes économiques, ou autres, qu'il devra respecter.

Par exemple, les composés TEKPol2 et TEKPol3 ci-dessous ont été synthétisés, et leur efficacité en DNP a été étudiée et comparée à celle du composé de référence bTbK.

### Synthèse des composés TEKPol2 et TEKPol3 :

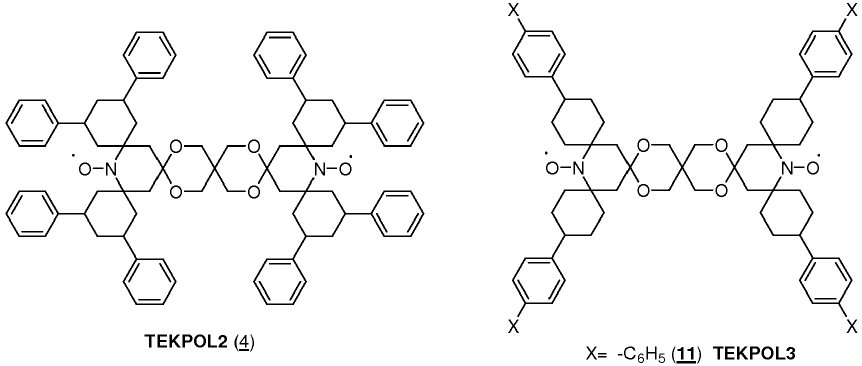

### Synthèse du composé TEKPol 2 (4)

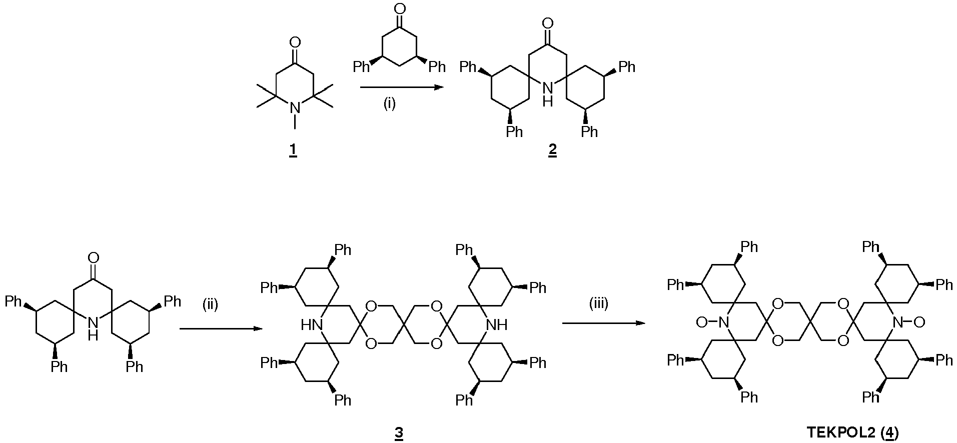

A un mélange de 1,2,2,6,6-pentaméthypipéridin-4-one **1** (2,00 g, 11,86 mmol) et de *cis-*3,5-diphénylcyclohexan-4-one (8,90 g, 35,60 mmol) dans le diméthylsulfoxyde (150 mL) a été ajouté du NH₄Cl (3,81g, 71,16 mmol) à température ambiante. Le mélange a été chauffé à 80°C pendant 16h, puis dilué avec 200 mL d'eau, et extrait avec 2 x 250 mL de chloroforme.

La phase organique a été concentrée sous pression réduite, diluée dans l'acétate d'éthyle (100 mL), lavée avec de la saumure (100 mL), séchée sur Na₂SO₄, et le solvant a été distillé sous pression réduite.

Le produit brut a été purifié par chromatographie sur colonne de silice avec comme solvant pentane/acétate d'éthyle (90/10) pour donner **2** (0,51g, 8%) sous la forme d'un solide blanc (en tant que mélange de diastéréoisomères).

¹H NMR (300 MHz, CDCl₃) *δ* 1.48-1.70 (m, 4H), 1.90-2.18 (m, 8H), 2.37-2.65 (m, 4H), 2.82-3.49 (m, 4H), 7.10-7.40 (m, 20H). ESI-MS *m*/*z* = 540 [M+H]⁺; 546 [M+Li]⁺.

Le composé **2** (0,45g, 0,83 mmol) a été dissous dans le toluène (80 mL), du pentaérythritol (51 mg, 0,38 mmol) et de l'acide p-toluènesulfonique (20 mg, 0,1 mmol) ont été ajoutés à la solution sous agitation. Le mélange a été chauffé à reflux dans un Dean Stark pendant 24h. Après refroidissement, la solution a été concentrée sous pression réduite et 100 mL d'une solution aqueuse à 10% de Na₂CO₃ ont été ajoutés. Le mélange a été extrait deux fois avec du chloroforme (150 mL), séché sur Na₂SO₄ et le solvant a été distillé sous pression réduite. Le résidu a été purifié par chromatographie sur colonne de silice avec un mélange CH₂Cl₂/EtOH (90/10) pour donner **3** (85 mg, 19%) sous la forme d'un solide blanc.

¹H NMR (CDCl₃) *δ* 1.50-2.20 (m, 32H), 2.70-3.28 (m, 8H), 3.50-3.92 (m, 8H), 7.00-7.56(m, 40H). ESI-MS *m*/*z* =1179 [M+H]⁺; 590 [M+2H]²⁺.

La diamine **3** (85 mg, 0,07 mmol) et Na₂WO₄.2H₂O (2 mg, 0,006 mmol) ont été mélangés sous agitation dans l'éthanol (5 mL) et du H₂O₂ (30%, 0,28 mmol, 32 µL) a été ajouté à 0°C. Le mélange a été agité à température ambiante pendant 4h, puis K₂CO₃ (0,10g) a été ajouté et la solution a été extraites deux fois avec du chloroforme (30 mL). La phase organique a été séchée sur Na₂SO₄ et distillée sous pression réduite. Le produit brut a été purifié par chromatographie sur colonne de silice en utilisant le chloroforme comme éluant pour donner le Tekpol 2 (**4**) pur sous la forme d'un solide rouge (39 mg, 45%).

Spectre RPE bande X (293 K, CH₂Cl₂): triplet, *A*_{N} = 1.48 mT. point de fusion: 230-233°C.ESI-MS *m*/*z* = 1209 [M+H]⁺; 1231 [M+Na] ⁺. HRMS-ESI calculé pour C₈₃H₈₈N₂O₆ ([M+NH₄]⁺) 1226.6981 trouvé 1226.6956. Analyse élémentaire: C, 82.04; H, 7.34; N, 2.32 calculé pour C₈₃H₈₈N₂O₆²· : C, 82.41; H, 7.33; N, 2.32.

### Synthèse du composé TEKPol 3 (11)

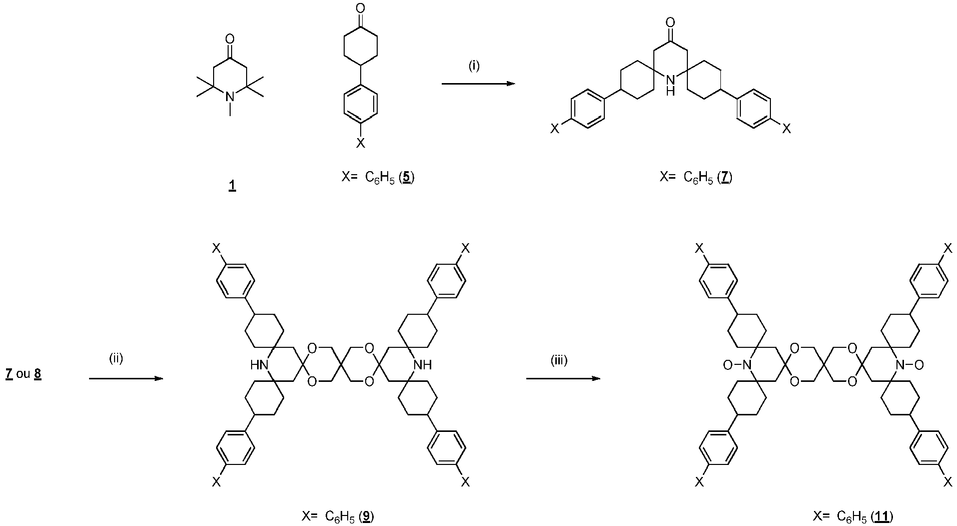

Le composé **7** a été synthétisé selon la procédure générale décrite ci-dessus pour le composé **2,** en utilisant de la 4-(4-biphénylyl)cyclohexanone (8,91 g, 35,65 mmol) au lieu de la *cis*-3,5-diphénylcyclohexan-4-one. Le produit brut a été purifié par chromatographie sur colonne de silice avec comme éluant pentane/acétate d'éthyle (90/10) pour donner **14** (0,18g, 3%) sous la forme d'un solide blanc.

¹H NMR (300 MHz, CDCl₃) *δ* 1.60-1.70 (m, 8H), 1.84-2.00 (m, 9H), 2.50-2.59 (m, 6H), 7.28-7.62 (m, 18H). ¹³C NMR (300 MHz, CDCl₃) *δ* 29.27, 40.22, 42.17, 48.07, 55.77, 125.99, 126.33, 127.69, 138.13, 139.99, 144.45, 209.81. ESI-MS *m*/*z* = 540 [M+H]⁺; 546 [M+Li]⁺.

A un mélange agité de 1,2,2,6,6-pentaméthylpipéridin-4-one **1** (0,88 g, 5,23 mmol) et de 4-(4-méthoxyphényl)cyclohexanone (3,20 g, 15,68 mmol) dans 80 mL de diméthylsulfoxyde, a été ajouté du NH₄Cl (1,68g, 31,38 mmol) à température ambiante. La mélange a été chauffé à 80°C pendant 16h, puis dilué avec 200 mL d'eau, extrait avec 2 x 250 mL de chloroforme. La phase organique a été concentrée sous pression réduite, diluée à l'acétate d'éthyle (100 mL), lavée avec de la saumure (100 mL), séchée sur Na₂SO₄, et le solvant a été distillé sous pression réduite. Le produit brut a été purifié par chromatographie sur colonne de silice avec comme éluant pentane/acétate d'éthyle (90/10) pour donner 8 (90 mg, 4%) sous la forme d'un solide blanc.

¹H NMR (300 MHz, CDCl₃) *δ* 1.44-2.00 (m, 17H), 2.40-2.55 (m, 6H), 3.78 (m, 6H), 6.83 (d, J = 8.34 Hz, 4H), 7.12 (d, J = 8.44 Hz, 4H). ¹³C NMR (300 MHz, CDCl₃) *δ* 30.50, 41.25, 42.62, 49.09, 55.26, 56.77, 113.81, 127.58, 138.56, 157.93, 211.02. ESI-MS *m*/*z* = 448 [M+H]⁺; 554 [M+Li]⁺.

La composé **7** (0,125 g, 0,23 mmol) a été dissous dans le toluène (40 mL). Du pentaérythritol (14 mg, 0,10 mmol) et de l'acide p-toluènesulfonique (5 mg, 0,03 mmol) ont été ajoutés à la solution sous agitation. Le mélange a été chauffé à reflux dans un Dean Stark pendant 24h. Après refroidissement, la solution a été concentrée sous pression réduite et 100 mL d'une solution aqueuse à 10% de Na₂CO₃ ont été ajoutés. Le mélange a été extrait deux fois avec du chloroforme (150 mL), séché sur Na₂SO₄, et le solvant a été distillé sous pression réduite. Le résidu a été purifié par chromatographie sur colonne de silice avec CH₂Cl₂/EtOH (90/10) pour donner **9** (30 mg, 25%) sous la forme d'un solide blanc.

¹H NMR (CDCl₃) *δ* 1.30-2.05 (m, 40H), 2.50 (m, 4H), 3.75 (s, 8H), 7.16-7.50 (m, 36H).¹³C NMR (CDCl₃) *δ* 30.63, 33.19, 37.84, 41.12, 43.19, 52.46, 63.72, 99.78, 126.97, 127.04, 127.13, 128.67, 138.94, 141.03, 145.90. ESI-MS *m*/*z* =1179 [M+H]⁺; 590 [M+2H]²⁺.

La diamine **9** (45 mg, 0,038 mmol) a été oxydée selon la procédure générale décrite ci-dessus pour le composé **4.** Le produit brut a été purifié par chromatographie sur colonne de silice avec le chloroforme comme éluant pour donner le Tekpol 3 (**11**) pur sous la forme d'un solide rouge pâle (21 mg, 45%).

Spectre RPE bande X (293 K, CH₂Cl₂): triplet, *A*_{N} = 1.51 mT. point de fusion: 275-278°C. ESI-MS *m*/*z* = 1209 [M+H]⁺; 1226 [M+NH₄]⁺. HRMS-ESI calculé pour C₈₃H₈₈N₂O₆·· ([M+H]⁺) 1209.6715 trouvé 1209.6720. Analyse élémentaire: C, 81.91; H, 7.57; N, 2.19 calculé pour C₈₃H₈₈N₂O₆·· : C, 82.41; H, 7.33; N, 2.32.

Le tableau 2 ci-dessous présente les valeurs d'efficacité DNP de ces deux composés selon l'invention, ainsi que celle du bPhCTbK dans les mêmes conditions expérimentales. A titre de comparaison, la valeur d'efficacité est également fournie pour le composé bTbK.

| Composé | ε_{C} |
|---|---|
| bPhCTbK | 200 |
| TEKPol2 | 222 |
| TEKPol3 | 166-250 |
| bTbK | 62 |

**Tableau 2 :** Facteur d'amplification (ε) du signal de RMN du 13C en abondance naturelle du tétrachloroéthane. Les conditions expérimentales sont les suivantes : tétrachloroéthane (10 mM), MAS ssNMR / DNP (100 K, 263 GHz, 9.4 T).

Les trois composés selon l'invention présentent un facteur d'amplification du signal RMN qui est nettement supérieur à celui du composé de référence, le bTbK.

### Références

1. Mak-Jurkauskas, M. L.; Griffin R.G., High-Frequency Dynamic Nuclear Polarization, in Solid-State NMR Studies of Biopolymers; McDermott, A. E. and Polenova T. Eds, Chp. 9, 2010, John Wiley& Sons, Ltd.
2. Barnes, A.B.; De Paépe, G.; van der Wel, P. C. A.; Hu, K.-N.; Joo C.-G.; Bajaj, V. S.; Mak-Jurkauskas, M. L.; Sirigiri, J. R.; Herzfeld, J.; Temkin, R. J.; Griffin R.G. App/. Magn. Reson. 2008, 34, 237-263.
3. Hu, K.-N., So/id State Nuc. Magn. Reson. 2011, 40, 31-41.
4. Themed Issue: Dynamic nuclear polarization, Phys. Chem. Chem. Phys. 2010, 12, 5725-5928.
5. Jannin, S.; Comment, A.; Kurdzesau, F.; Konter, J. A.; Hautle, P.; v. d. Brandt, B.; v. d. Klink J. J. J. Chem. Phys. 2008, 128, 241102.
6. Lumata, L.; Jindal, A. K.; Merritt, M. E.; Maloy, C. R.; Sherry, A. D.; Kovacs, Z. J. Am. Chem. Soc. 2011, 133, 8673.
7. Smith, A. A.; Corzilius, B.; Barnes, B.; Maly, T.; Griffin, R. G. J. Chem. Phys. 2012, 136, 015101.
8. Hu, K.-N.; Yu, H.; Swager T.; Griffin, R. G., J. Am. Chem. Soc. 2004, 126, 10844.
9. Song, C.; Hu, K.-N.; Joo C.-G.; Swager T.; Griffin, R. G., J. Am. Chem. Soc. 2006, 128, 11385.
10. Maly, T.; Debelouchina, G. T.; Bajaj, V. S.; Hu, K.-N.; Joo, C.-G.; Mak-Jurkauskas, M. L.; Sirigiri, J. R.; van der Wel, P. C.; Henfeld, J.; Temkin, R. J.; Griffin, R.G. J. Chem. Phys., 2008, 128, 052211.
11. Hu, K.-N.; Song, c.; Yu, H.; Swager, T. M.; Griffin, R. G. J. Chem. Phys. zoos, 128, 052302.
12. Matsuki, Y.; Maly, T.; Ouari, 0.; Karoui, H.; Le Moigne, F.' Rizzato, E.; Lyubenova, S.; Herzfeld, J.; Prisner, T.; Tordo, P.; Griffin, R. G. Angew. Chem. nt. Ed 2009, 48, 4996.
13.Ysacco, C.; Rizzato, E.; Virolleaud, M. A.; Karoui, H.; Rockenbauer, A.; Le Moigne, F.; Ouari, O; Griffin, R. G.; Tordo, P. Phys. Chem. Chem. Phys. 2010, 12, S841.
14. Karoui, H. ; Le Moigne, F. ; Ouari, 0.; Tordo, P. ; Nitroxide Radicals: Properties, Synthesis and Applications, in Stable Radicals, Hicks, R. G. ed., Chp. 5, pp 173-220, 2010, John Wiley& Sons, Ltd.
15.Wind, R. A.; Ardenkjaer-Larsen J.-H., J. Magn. Reson. 1999, 141, 347.
16.Wolber, J.; Ellner, F.; Fridlund, B.; Gram, A.; Johannesson, H.; Hansson, G.; Hansson, L. H.; Lerche, M. H.; Mansson, S.; Sen/in, R.; Thaning, M.; Golman, K.; Ardenkjaer-Larsen J.-H. Nucl. Instrum. Methods Phys. Res., Sect. A, 2004, 526, 173.
17. Ardenkjaer-Larsen J.-H.; Fridlund, B.; Gram, A.; Hansson, G.; Hansson, L. H.; Lerche, M. H.; Senlin, R.; Thaning, M.; Golman, K. Proc. Natl. Acad. Sci. USA, 2003, 100, 10158.
18. Lumata, L.; Ratnakar, S. J.; Jindal, A.; Merritt, M.; Comment, A.; Malloy, G.; Sherry, A. D.; Kovacs, Z. Chem. Eur. J. 2011, 17, 10825.
19.(a) Dane, E. L.; Swager, T. M., J. Org. Chem., 2010, 75, 3533-3536. (b) Haze, 0.; Oorzilius, B.; Smith, A. A.; Griffin, R. G.; Swager, T. M. J. Am. Chem. Soc. 2012, 134, 14287.
20. Dane, E. L.; Corzilius, B.; Rizzato, E.; Stocker, P.; Maly, T.; Smith, A. A.; Griffin, R. G.; Ouari, 0.; Tordo, P.; Swager, T. M. J. Org. Chem. 2012, 77, 1789.
21.Ysacco, C.; Karoui, H.; Casano, G.; Le Moigne, F.; Combes, S.; Rockenbauer, A.; Rosay, M.; Werner, M.; Ouari, 0.; Tordo, P., App/. Magn. Resan. 2012, 43, 251.
22.Thurber, R. K.; Yau, W.-M; Ticko, R., J. Magn. Reson. 2010, 204, 303.
23.Gafurov, M.; Lyubenova, S.; Denysenko, V.; Ouari, 0.; Karoui, H.; Le Moigne, F.; Tordo, P.; Prisner, T. Appl. Magn. Reson. 2010, 37, 505.
24. Zagdoun, A.; Casano, G.; Ouari, 0.; Lapadula, G.; Rossini, A. J.; Lelli, M.; Baffert, M.; Gajan, D.; Veyre, L.; Maas, W. E.; Rosay, M.; Weber, R. T.; Thieuleux, C.; Coperet, C.; Lesage, A.; Tordo, P.; Emsley, L., J. Am. Chem. Soc., 2012, 134, 2284.
25. Dane, E. L.; Maly, T.; Debelouchina, G. T.; Griffin, R. G.; Swager, T. M. Org. Lett. 2009, 9, 1871.
26.Liu, Y. P.; Villamena, F. A.; Song, Y. G.; Sun, J. A.; Rockenbauer, A.; Zweier, J. L. J. Org. Chem. 2010, 75, 7796.
27. Hu, K.-N.; Bajaj, V. S.; Rosay, M.; Griffin, R. G. J. Chem. Phys. 2007, 126, 044512.
28.a) Debelouchina, 6. T.; Platt, G. w.; Bayro, M. J.; Radford, s. E.; Griffin, R. c-1., J. Am. Chem. Soc. 2011, 133, 17077. b) Bayro, M. J.; Debelouchina, G. T.; Eddy, M. T.; Birkett, N. R.; MacPhee, C. E.; Rosay, M.; Maas, W. E.; Dobson, C. M.; Griffin, R. G. J. Am. Chem. Soc. 2011, 133) 13967.
29.a) Salnikov, E.; Rosay, ; Pawsey, ; Ouari, 0.; Tordo P.; Bechinger, B. J. Am. Chem. 50c. 2010, 132, 5940. b) Linden, A. H.; Lange, S.; Franks, W. T.; Akbey, U.; Specker, E.; van Rossum, B.-J.; Oschkinat, H. J. Am. Chem. Soc. 2011, 133, 19266. c) Jacso, T.; Franks, W. T.; Rose, H.; Fink, U.; Broecker, J.; Keller, S.; Oschkinat, H.; Reif, B. Angew. Chem. Int. Ed. 2012, 51, 432. d) Reggie, L.; Lopez, J. J.; Collinson, I.; C. Glaubitz, C.; Lorch M. J. Am. Chem. Soc. 2012, 134, 19084.
30. Sergeyev, I. V.; Day, L. A.; Goldbourt, A.; McDermott, A. E. J. Am. Chem. Soc. 2011, 133, 20208.
31. Renault, M.; Pawsey, S.; Bos, M. P.; Koers, E. J.; Nand, D.; Tommassen-van Boxtel, R.; Rosay, M.; Tommassen, J.; Maas, W. E.; Baldus, M.; Angew. Chem. Int. Ed. 2012, 51, 2998.
32. Lelli, M.; Gajan, D.; Lesage, A.; Caporini, M. A.; Vitzthum, V.; Mieville, P.; Heroguel, F.; Rascon, F.; Roussey, A.; Thieuleux, C.; Boualleg, M.; Veyre, L.; Bodenhausen, G.; Coperet, C.; Emsley, L. J. Am. Chem. Soc. 2011, 133, 2104.
33. Zagdoun, A.; Rossini, A. J.; Gajan, D.; Bourdolle, A.; Ouari, 0.; Rosay, M.; Maas, W. E.; P.Tordo; Lelli, M. Emsley, L.; Lesage, A.; Copéret, C. Chem. Commun. 2012, 48, 654.
34. Rossini, A. J.; Zagdoun, A.; Lelli, M.; Canivet, J.; Aguado, 0.; Ouari, 0.; P.Tordo; Rosay, M.; Maas, W. E.; Copéret, C.; Farusseng, D.; Emsley, L.; Lesage, A. Angew. Chem. Int. Ed., 2012, 51, 123.
35.a) Vitzthum, V.; Mieville, P.; Carnevale, D.; Caporini, M. A.; Gajan, D.; Copéret, C.; Lelli, M.; Zagdoun, A.; Rossini, A. J.; Lesage, A.; Emsley, L.; Bodenhausen, G. Chem. Commun. 2012, 48, 1988. b) Lee, D.; Takahashi, H.; Thankamony, A. S. L.; Dacquin, J.-P.; Bardet, M.; Lafon, 0.; De Paepe, G. J. Am. Chem. Soc. 2012, 134 18491.
36. Blanc, F.; Sperrin, L.; Jefferson, A. D.; Pawsey, S.; Rosay, M.; Grey, P. C. J. Am. Chem. Soc. 2013, dx.doi.org/10.1021/ja4004377
37.a) Lafon, 0.; Thankamony, A. S. L.; Rosay, M.; Aussenac, F.; Lu, X.; Trébosc, J.; Bout-Roumazeilles, H.; Vezin, H.; Amoureux, J.-P. Chem. Commun. 2013, .' D01: 10.1039/C2CC36170A. b) Lafon, 0.; Thankamony, A. S. L.; Kobayashi T.; Carnevale, D.; Vitzthum, V.; Slowing, I. 1.; Kandel, K.; Vezin, H.; Amoureux, J.-P.; Bodenhausen, G. J. Chem. Phys. C 2013, D0I: 10.1021/1123101095.
38.a) A. J. Rossini, A. Zagdoun, F. Hegner, M. Schwarzwalder, D. Gajan, C. Coperet, A. Lesage, L. Emsley, J. Am. Chem. Soc. 2012, 134, 16899. b) H. Takahashi, D. Lee, L. Dubois, M. Bardet, S. Hediger, G. De Paepe, Angewandte Chemie International Edition 2012, 51, 11766.
39. A. J. Rossini, A. Zagdoun, M. Lelli, A. Lesage, C. Coperet, L. Emsley, Acc. Chem. Res. 2013

## Revendications

1. Composé biradicalaire du type dinitroxyde, de formule générale : 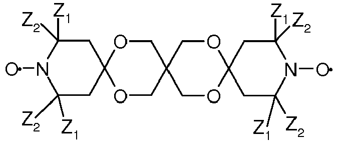 dans laquelle
- Z1 et Z2, combinés ensemble avec le même atome de carbone du cycle nitroxyde auquel ils sont liés, forment un spirocyclohexyle substitué par un ou deux groupements R3, R3 étant un groupement phényle éventuellement substitué par un ou plusieurs groupements R4, R4 étant un phényle.

2. Composé biradicalaire de type dinitroxyde selon la revendication 1, **caractérisé en ce qu'**il est marqué isotopiquement par l'un des marqueurs choisis parmi le Deutérium (²H), le Carbone 13 (¹³C) et l'Azote 15 (¹⁵N).

3. Composé biradicalaire de type dinitroxyde selon l'une des revendications 1 et 2, étant le bPhCTbK (bis-PhénylCyclohexylTEMPO-bis-Ketal), c'est-à-dire le 22,41-dinitroxyl-3,19,26,38-tetraphenyl-22,41 diazaheptaspiro [5.1.2.2.1.5.1.5.1. 2.2.1. 5.1] hentetracontane, facultativement substitué sur un ou plusieurs des groupements phényles, le 22,41-dinitroxyl-2,4,18,20,25,27,37,39-octaphenyl-22,41 diazaheptaspiro [5.1.2.2.1.5.1.5.1.2.2.1.5.1] hentetracontane facultativement substitué sur un ou plusieurs des groupements phényles ou le 22,41-dinitroxyl-3,19,26,38-tetra-p-biphenyl-22,41 diazaheptaspiro [5.1.2.2.1.5.1.5.1.2.2.1.5.1] hentetracontane facultativement substitué sur un ou plusieurs des groupements phényles.

4. Utilisation d'un composé biradicalaire du type dinitroxyde selon l'une des revendications 1 à 3 comme agent de polarisation pour la Résonance Magnétique Nucléaire dans les techniques de physique des particules ainsi que dans les techniques d'imagerie médicale.

5. Composition comprenant au moins un composé biradicalaire de type dinitroxyde défini selon l'une des revendications 1 à 4.

6. Procédé de polarisation d'un échantillon comprenant une étape pour mettre en contact l'échantillon avec un agent de polarisation, **caractérisé en ce que** l'agent de polarisation est un composé biradicalaire de type dinitroxyde selon l'une des revendications 1 à 4.

7. Procédé d'analyse de Résonance Magnétique Nucléaire, de physique des particules ou d'imagerie médicale, comprenant une polarisation d'un échantillon dans laquelle l'échantillon est mis en contact avec un agent de polarisation, **caractérisé en ce que** l'agent de polarisation est un composé biradicalaire de type dinitroxyde selon l'une des revendications 1 à 4.

## Patentansprüche

1. Biradikale Dinitroxidverbindung der allgemeinen Formel: 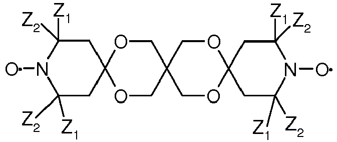 in der
- Z1 und Z2 zusammen mit demselben Kohlenstoffatom des Nitroxidrings, an das sie gebunden sind, ein Spirocyclohexyl bilden, das mit einer oder zwei Gruppen R3 substituiert ist, wobei R3 eine Phenylgruppe ist, die eventuell mit einer oder mehreren Gruppen R4 substituiert ist, wobei R4 ein Phenyl ist.

2. Biradikale Dinitroxidverbindung gemäß Anspruch 1, **dadurch gekennzeichnet, dass** sie mit einem Marker isotopenmarkiert ist, der aus den Markern Deuterium (²H), Kohlenstoff 13 (¹³C) und Stickstoff 15 (¹⁵N) ausgewählt ist.

3. Biradikale Dinitroxidverbindung gemäß einem der Ansprüche 1 und 2, wobei bPhCTbK (bis-PhenylCyclohexylTEMPO-bis-Ketal), d.h. 22,41-Dinitroxyl-3,19,26,38-tetraphenyl-22,41-diazaheptaspiro-[5.1.2.2.1.5.1.5.1.2.2.1.5.1]-hentetracontan, gegebenenfalls substituiert an einer oder mehreren Phenylgruppen, 22,41-Dinitroxyl-2,4,18,20,25,27,37,39-octaphenyl-22,41-diazaheptaspiro-[5.1.2.2.1.5.1.5.1.2.2.1.5.1]-hentetracontan, gegebenenfalls substituiert an einer oder mehreren Phenylgruppen, oder 22,41-Dinitroxyl-3,19,26,38-tetra-*p*-biphenyl-22,41-diazaheptaspiro-[5.1.2.2.1.5.1.5.1.2.2.1.5.1]-hentetracontan, gegebenenfalls substituiert an einer oder mehreren Phenylgruppen, ist.

4. Verwendung einer biradikalen Dinitroxidverbindung gemäß einem der Ansprüche 1 bis 3 als Polarisationsmittel für die Magnetische Kernresonanz in den Techniken der Teilchenphysik sowie in den Techniken der medizinischen Bildgebung.

5. Zusammensetzung, umfassend wenigstens eine biradikale Dinitroxidverbindung, definiert in einem der Ansprüche 1 bis 4.

6. Verfahren zur Polarisation einer Probe, umfassend einen Schritt des Inkontaktbringens der Probe mit einem Polarisationsmittel, **dadurch gekennzeichnet, dass** das Polarisationsmittel eine biradikale Dinitroxidverbindung gemäß einem der Ansprüche 1 bis 4 ist.

7. Verfahren zur Analyse der Magnetischen Kernresonanz, der Teilchenphysik oder der medizinischen Bildgebung, umfassend eine Polarisation einer Probe, wobei die Probe mit einem Polarisationsmittel in Kontakt gebracht wird, **dadurch gekennzeichnet, dass** das Polarisationsmittel eine biradikale Dinitroxidverbindung gemäß einem der Ansprüche 1 bis 4 ist.

## Claims

1. A biradical compound of the dinitroxide type, of general formula: 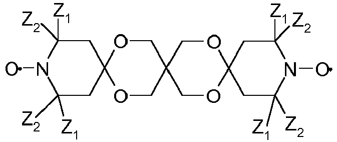 wherein
- Z1 and Z2, combined together with the same carbon atom of the nitroxide ring to which they are bound, form a spirocyclohexyl substituted with one or two groups R3, R3 being a phenyl group optionally substituted with one or more groups R4, R4 being a phenyl.

2. The biradical compound of the dinitroxide type according to claim 1, **characterized in that** it is labelled isotopically with one from among deuterium (²H), carbon 13 (¹³C) and nitrogen 15 (¹⁵N).

3. The biradical compound of the dinitroxide type according to claim 1 or 2, being bPhCTbK (bis-PhenylCyclohexylTEMPO-bis-Ketal), i.e. 22,41-dinitroxyl-3,19,26,38-tetraphenyl-22,41 diazaheptaspiro [5.1.2.2.1.5.1.5.1.2.2.1.5.1] hentetracontane, optionally substituted on one or more of the phenyl groups, 22,41-dinitroxyl-2,4,18,20,25,27,37,39-octaphenyl-22,41 diazaheptaspiro [5.1.2.2.1.5.1.5.1.2.2.1.5.1] hentetracontane optionally substituted on one or more of the phenyl groups or 22,41-dinitroxyl-3,19,26,38-tetra-p-biphenyl-22,41 diazaheptaspiro [5.1.2.2.1.5.1.5.1.2.2.1.5.1] hentetracontane optionally substituted on one or more of the phenyl groups.

4. Use of a biradical compound of the dinitroxide type according to one of claims 1 to 3, as polarizing agent for the Nuclear Magnetic Resonance in particle physics techniques as well as in medical imaging techniques.

5. A composition comprising at least one biradical compound of the dinitroxide type defined according to one of claims 1 to 4.

6. A process of polarization of a sample comprising a step for bringing the sample into contact with a polarizing agent, **characterized in that** the polarizing agent is a biradical compound of the dinitroxide type according to one of claims 1 to 4.

7. A process of Nuclear Magnetic Resonance analysis, particle physics or medical imaging, comprising polarization of a sample in which the sample is brought into contact with a polarizing agent, **characterized in that** the polarizing agent is a biradical compound of the dinitroxide type according to one of claims 1 to 4.
